# EUROPEAN PATENT APPLICATION

(11) **EP 3 015 071 A1**
(43) Date of publication of application: **04.05.2016**
(21) Application number: 15163471.4
(22) Date of filing: 14.04.2015
(51) Int. Cl.: A61B 8/00

(54) **ULTRASOUND APPARATUS AND INFORMATION INPUT METHOD THEREOF**

(30) Priority: 27.10.2014 KR 20140146425
(71) Applicant: Samsung Medison Co., Ltd., Hongcheon-gun, Gangwon-do 250-875 (KR)
(72) Inventor: Lee, Seung-ju, Gangwon-do (KR); Jun, Yoon-woo, Seoul (KR)
(74) Representative: D'Halleweyn, Nele Veerle Trees Gertrudis

(57) **Abstract**

Disclosed is an ultrasound apparatus. The ultrasound apparatus includes a user input unit that receives a user input which selects a menu for setting an annotation in a first ultrasound image displayed on a screen of the ultrasound apparatus, a control unit that determines a photographing part of an object in the first ultrasound image, and a display unit that displays a setting window including at least one recommendation annotation among a plurality of annotations corresponding to the determined photographing part of the object. The user input unit receives a user input which selects one from the at least one recommendation annotation, and the display unit displays an image, representing the selected recommendation annotation, on the first ultrasound image. The at least one recommendation annotation is an annotation which is previously input by the user and is generated in the ultrasound apparatus in correspondence with a second ultrasound image including the photographing part.

## Description

### RELATED APPLICATION

This application claims the benefit of Korean Patent Application No. 10-2014-0146425, filed on October 27, 2014, in the Korean Intellectual Property Office, the disclosure of which is incorporated herein in its entirety by reference.

### BACKGROUND

### 1. Field

One or more exemplary embodiments relate to an information input method and an ultrasound apparatus therefor, which provide recommendation data, based on a context of a user about the ultrasound apparatus.

### 2. Description of the Related Art

Ultrasound apparatuses transmit ultrasound signals generated by transducers of a probe to an object and receive echo signals reflected from the object, thereby obtaining at least one image of an internal part of the object. In particular, ultrasound apparatuses are used for medical purposes including observation of the interior of an object, detection of foreign substances, and diagnosis of damage to the object. Such ultrasound apparatuses provide high stability, display images in real time, and are safe due to the lack of radioactive exposure, compared to X-ray apparatuses. Therefore, ultrasound apparatuses are widely used together with other image diagnosis apparatuses.

Ultrasound apparatuses transmit ultrasound signals generated by transducers of a probe to an object and receive echo signals reflected from the object, thereby obtaining at least one image of an internal part of the object. In particular, ultrasound apparatuses are used for medical purposes including observation of the interior of an object, detection of foreign substances, and diagnosis of damage to the object. Such ultrasound apparatuses provide high stability, display images in real time, and are safe due to the lack of radioactive exposure, compared to X-ray apparatuses. Therefore, ultrasound apparatuses are widely used together with other image diagnosis apparatuses including a computed tomography (CT) apparatus, a magnetic resonance imaging (MRI) apparatus, and the like.

A user often inputs an annotation of an object which is shown in an ultrasound image. For example, a user often marks a name, a shape, or a region of interest (ROI) of an organ which is shown in an ultrasound image. Also, there may be an annotation which is frequently used by a number of users for the same photographing part. Also, depending on a user or a hospital, only some annotations may be frequently used for the same photographing part.

Therefore, it is required to develop a user interface which provides an annotation necessary for a user depending on a diagnosis situation without inputting an annotation word by word by using a keyboard each time the user inputs the annotation.

### SUMMARY

One or more exemplary embodiments include an ultrasound image processing method and an ultrasound apparatus therefor, which provide recommendation data, based on a context of a user about the ultrasound apparatus.

Additional aspects will be set forth in part in the description which follows and, in part, will be apparent from the description, or may be learned by practice of the presented exemplary embodiments.

According to one or more exemplary embodiments, an ultrasound apparatus includes: a user input unit that receives a user input which selects a menu for setting an annotation in a first ultrasound image displayed on a screen of the ultrasound apparatus; a control unit that determines a photographing part of an object in the first ultrasound image; and a display unit that displays a setting window including at least one recommendation annotation among a plurality of annotations corresponding to the determined photographing part of the object, wherein, the user input unit receives a user input which selects one from the at least one recommendation annotation, and the display unit displays an image, representing the selected recommendation annotation, on the first ultrasound image, and the at least one recommendation annotation is an annotation which is previously input by the user and is generated in the ultrasound apparatus in correspondence with a second ultrasound image including the photographing part.

The at least one recommendation annotation may include different kinds of annotations.

The at least one recommendation annotation may be an annotation which is previously set in the ultrasound apparatus by the user in correspondence with the photographing part.

The at least one recommendation annotation may include at least one selected from a phrase, a body marker, and an arrow.

The ultrasound apparatus may further include a storage unit that stores an order of the plurality of annotations with respect to the number of settings, wherein the at least one recommendation annotation may be an annotation, in which the order is within a predetermined order, among the plurality of annotations.

The ultrasound apparatus may further include a storage unit that stores an order of the plurality of annotations with respect to an order in which the plurality of annotations are generated in the ultrasound apparatus, wherein the at least one recommendation annotation may be an annotation, in which the order is within a predetermined order, among the plurality of annotations.

The setting window may include a screen keyboard that includes a plurality of keys.

The setting window may include a movement display button that switches the setting window to a screen keyboard including a plurality of keys, and when a user input which selects the movement display button is received, the display unit may switch the setting window to the screen keyboard and displays the screen keyboard.

The first ultrasound image may include two different ultrasound images, and the setting window may include two setting windows respectively corresponding to the two different ultrasound images.

The control unit may acquire at least one recommendation annotation which is pre-stored in correspondence with the photographing part, based on at least one selected from identification information of the object and a user of the ultrasound apparatus, and the display unit may display a setting window including the acquired at least one recommendation annotation.

According to one or more exemplary embodiments, an information input method includes: receiving a user input which selects a menu for setting an annotation in a first ultrasound image displayed on a screen of an ultrasound apparatus; determining a photographing part of an object in the first ultrasound image; displaying a setting window including at least one recommendation annotation among a plurality of annotations corresponding to the determined photographing part of the object; receiving a user input which selects one from the at least one recommendation annotation; and displaying an image, representing the selected recommendation annotation, on the first ultrasound image, wherein, the at least one recommendation annotation is an annotation which is previously input by the user and is generated in the ultrasound apparatus in correspondence with a second ultrasound image including the photographing part.

The setting window may include a movement display button that switches the setting window to a screen keyboard including a plurality of keys, and the information input method may further include, when a user input which selects the movement display button is received, switching the setting window to the screen keyboard and displaying the screen keyboard.

The displaying of the setting window may include: acquiring at least one recommendation annotation which is pre-stored in correspondence with the photographing part, based on at least one selected from identification information of the object and a user of the ultrasound apparatus; and displaying a setting window including the acquired at least one recommendation annotation.

### BRIEF DESCRIPTION OF THE DRAWINGS

These and/or other aspects will become apparent and more readily appreciated from the following description of the exemplary embodiments, taken in conjunction with the accompanying drawings in which:
FIG. 1 is a block diagram of an ultrasound apparatus according to an exemplary embodiment;
FIG. 2 is a diagram illustrating a method in which an ultrasound apparatus provides a screen keyboard for inputting an annotation onto an ultrasound image, according to an exemplary embodiment;
FIG. 3A is a flowchart for describing a method in which an ultrasound apparatus sets an annotation in an ultrasound image, according to an exemplary embodiment;
FIG. 3B is a diagram for describing a database storing a recommendation annotation, according to an exemplary embodiment;
FIG. 4 is a diagram for describing a method in which an ultrasound apparatus provides a recommendation annotation on the basis of a photographing part, according to an exemplary embodiment;
FIGS. 5A and 5B are diagrams for describing a method in which an ultrasound apparatus provides a recommendation annotation on the basis of a photographing part, according to another exemplary embodiment;
FIG. 6 is a diagram for describing a method in which an ultrasound apparatus provides a screen keyboard, according to an exemplary embodiment;
FIG. 7 is a diagram for describing a method in which an ultrasound apparatus provides a recommendation annotation image, according to an exemplary embodiment;
FIG. 8 is a diagram for describing a method in which an ultrasound apparatus provides an interface for generating an annotation image, according to an exemplary embodiment;
FIG. 9 is a diagram for describing a method in which an ultrasound apparatus provides a setting window corresponding to a plurality of ultrasound images, according to an exemplary embodiment;
FIG. 10 is a diagram for describing a method in which an ultrasound apparatus provides recommendation data corresponding to an input field, according to an exemplary embodiment; and
FIG. 11 is a block diagram of an ultrasound apparatus according to another exemplary embodiment.

### DETAILED DESCRIPTION

Reference will now be made in detail to exemplary embodiments, examples of which are illustrated in the accompanying drawings, wherein like reference numerals refer to like elements throughout. In this regard, the present exemplary embodiments may have different forms and should not be construed as being limited to the descriptions set forth herein. Accordingly, the exemplary embodiments are merely described below, by referring to the figures, to explain aspects of the present description.

Hereinafter, the terms used in the specification will be briefly defined, and the embodiments will be described in detail.

The terms used in this specification are those general terms currently widely used in the art in consideration of functions regarding the present invention, but the terms may vary according to the intention of those of ordinary skill in the art, precedents, or new technology in the art. Also, some terms may be arbitrarily selected by the applicant, and in this case, the meaning of the selected terms will be described in detail in the detailed description of the present specification. Thus, the terms used herein have to be defined based on the meaning of the terms together with the description throughout the specification.

When a part "includes" or "comprises" an element, unless there is a particular description contrary thereto, the part can further include other elements, not excluding the other elements. In addition, terms such as "... unit", "... module", or the like refer to units that perform at least one function or operation, and the units may be implemented as hardware or software or as a combination of hardware and software.

Reference will now be made in detail to embodiments, examples of which are illustrated in the accompanying drawings. In this regard, the present embodiments may have different forms and should not be construed as being limited to the descriptions set forth herein. In the accompanying drawings, a portion irrelevant to a description of the inventive concept will be omitted for clarity. Moreover, like reference numerals refer to like elements throughout.

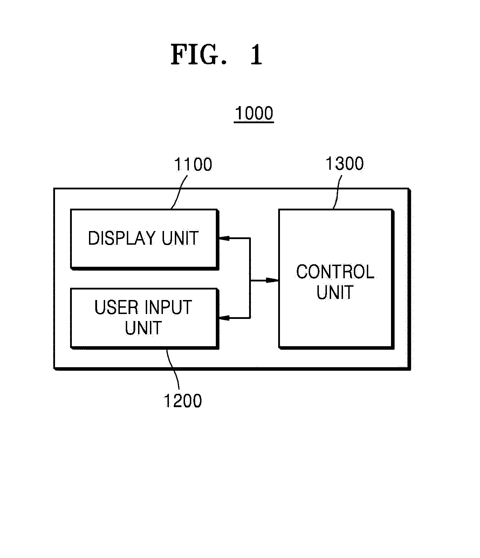

FIG. 1 is a block diagram of an ultrasound apparatus 1000 according to an exemplary embodiment.

Referring to FIG. 1, the ultrasound apparatus 1000 may include a display unit 1100, a user input unit 1200, and a control unit 1300.

The display unit 1100 may display information which is to be supplied to a user.

For example, the display unit 1100 may display an ultrasound image. The display unit 1100 may display an ultrasound image captured by the ultrasound apparatus 1000 and display an ultrasound image received from an external device.

Moreover, the display unit 1100 may display a menu for setting an annotation in the ultrasound image. Also, as a user input which selects the menu for setting the annotation in the ultrasound image is received, the display unit 1100 may display a setting window including at least one annotation which is pre-stored in correspondence with a photographing part. Also, as a user input which selects one annotation from among at least one or more annotations is received, the display unit 1100 may display an image, representing a selected annotation, in the ultrasound image.

The user input unit 1200 may be an input device for inputting data, a command, or a request to the ultrasound apparatus 1000.

For example, the user input unit 1200 may receive a user input which selects a menu for setting an annotation in an ultrasound image.

Moreover, the user input unit 1200 may receive a user input which selects one annotation from among at least one or more annotations corresponding to a photographing part which is displayed on a screen.

The control unit 1300 may overall control the elements of the ultrasound apparatus 1000. The control unit 1300 may control the display unit 1100 and the user input unit 1200.

Moreover, the control unit 1300 may determine a photographing part of an object in the ultrasound image. Also, the control unit 1300 may acquire at least one recommendation annotation which is pre-stored in correspondence with the photographing part.

The at least one recommendation annotation, which is pre-stored in correspondence with the photographing part, may be an annotation which is pre-generated in the ultrasound apparatus 1000 by a user in correspondence with the ultrasound image including the photographing part. The at least one recommendation annotation, which is pre-stored in correspondence with the photographing part, may be an annotation which is preset in the ultrasound apparatus 1000 by the user in correspondence with the photographing part. Also, the at least one recommendation annotation may include at least one selected from a phrase, a body marker, and an arrow.

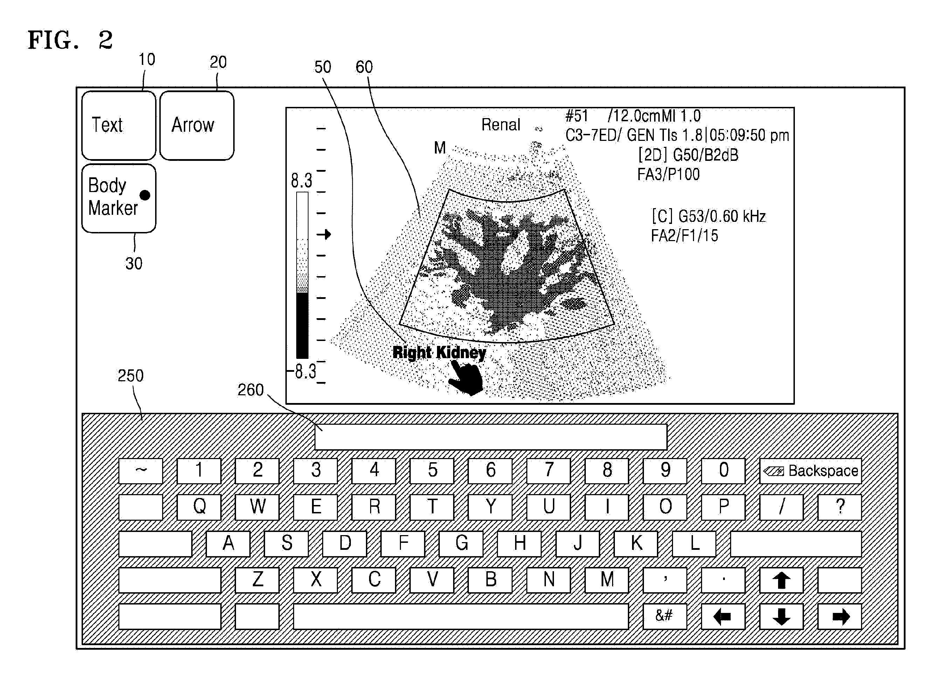

FIG. 2 is a diagram illustrating a method in which the ultrasound apparatus 1000 provides a screen keyboard 250 for inputting an annotation onto an ultrasound image, according to an exemplary embodiment.

Referring to FIG. 2, the ultrasound apparatus 1000 may provide a plurality of buttons 10, 20 and 30 for setting an annotation in an ultrasound image 60 which is displayed on a screen.

The buttons 10, 20 and 30 for setting the annotation in the ultrasound image may include a text button 10 for setting text information, an arrow button 20 for setting an arrow, and a body marker button 30 for setting a body marker.

As a user input which selects the text button 10 for setting text information is received, the ultrasound apparatus 1000 may display the screen keyboard 250 on a screen. The screen keyboard 250 may include a number key, a letter key, a sign key, and a function key.

As a user input which selects at least one key included in the screen keyboard 250 is received, the ultrasound apparatus 1000 may display a key value of at least one selected key on a text box 260. Also, as a user input which selects a predetermined key (for example, an enter key) of the screen keyboard 250 is received, the ultrasound apparatus 1000 may display at least one key value, input to the text bot 260, on an ultrasound image 60. Therefore, a user may set a text annotation, intended by the user, in the ultrasound image 60.

Moreover, as a user input which selects and moves text information displayed on the ultrasound image 60 is received, the ultrasound apparatus 1000 may move a position of the text information in the ultrasound image 60.

Moreover, when a user input which selects the arrow button 20 for setting an arrow is received, the ultrasound apparatus 1000 may display various kinds of pre-stored arrow images. Also, when a user input which selects the body marker button 30 for setting a body marker is received, the ultrasound apparatus 1000 may display various kinds of pre-stored body markers. As a user input which selects one from among a displayed arrow image and a displayed body marker is received, the ultrasound apparatus 1000 may display the selected arrow image or body marker on the ultrasound image 60.

Moreover, as a user input which sets an annotation in the ultrasound image 60 is received, the ultrasound apparatus 1000 may store the set annotation. Also, the ultrasound apparatus 1000 may store a date at which the set annotation is stored. Also, the ultrasound apparatus 1000 may calculate the number of times the set annotation is stored. Therefore, the ultrasound apparatus 1000 may calculate a user annotation which is latest set or the order of an annotation which is the most used for a certain time, and provide the calculated annotation as recommendation annotations to the user.

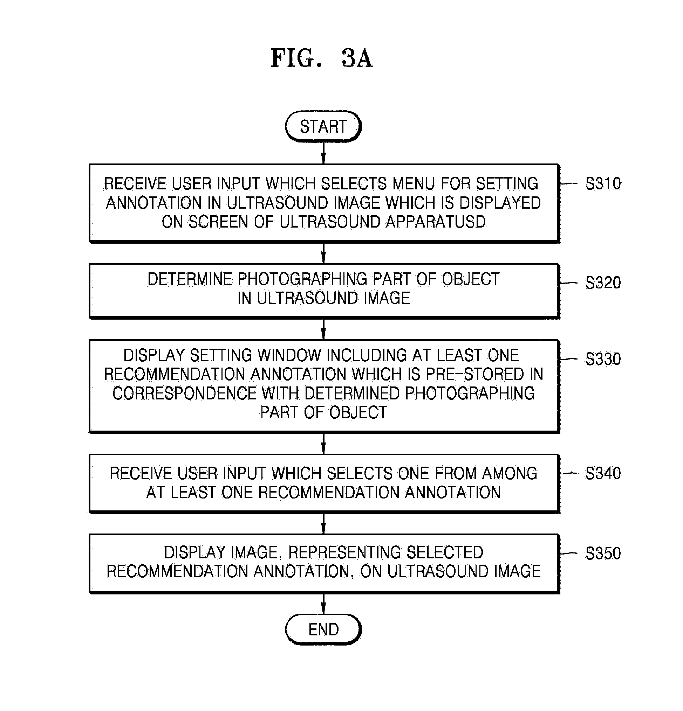

FIG. 3A is a flowchart for describing a method in which the ultrasound apparatus 1000 sets an annotation in an ultrasound image, according to an exemplary embodiment.

In operation S310, the ultrasound apparatus 1000 may receive a user input which selects a menu for setting an annotation in a first ultrasound image which is displayed on a screen of the ultrasound apparatus 1000.

The ultrasound apparatus 1000 may display the first ultrasound image. For example, the first ultrasound image may be at least one selected from a brightness (B) mode image in which a level of an ultrasound echo signal reflected from an object is expressed as brightness, a color (C) mode image in which a speed of a moving object is expressed as a color by using the Doppler effect, a Doppler (D) mode image in which an image of a moving object is expressed as a spectrum type by using the Doppler effect, a motion (M) mode image that shows a motion of an object with time at a certain place, and an elastic mode image in which a reaction difference between when compression is applied to an object and when compression is not applied to the object is expressed as an image, but is not limited thereto. Also, the first ultrasound image may be a two-dimensional (2D) image, a three-dimensional (3D) image, or a four-dimensional (4D) image. The ultrasound apparatus 1000 may photograph an object to acquire the first ultrasound image. Also, the ultrasound apparatus 1000 may receive the first ultrasound image from an external device.

The ultrasound apparatus 1000 may display a menu for setting an annotation in the first ultrasound image. Also, the ultrasound apparatus 1000 may receive a user input which selects a menu for setting an annotation in the first ultrasound image which is displayed on the screen of the ultrasound apparatus 1000.

An annotation may denote a phrase or an image which is set in the first ultrasound image by a user. The annotation may be information which explains an object in the first ultrasound image. For example, the annotation may be information which explains an organ in the first ultrasound image. Also, the annotation may be information which explains the user, the object itself, or the ultrasound apparatus 1000.

In operation S320, the ultrasound apparatus 1000 may determine a photographing part of the object in the first ultrasound image.

The photographing part of the object may be set in the ultrasound apparatus 1000 by the user. For example, the user may set a photographing part, which is to be photographed, before photographing the object. A plurality of photographing parts may have different depths from a skin, different sizes, or different tissue types. Therefore, the photographing parts may have different parameter values such as ultrasound energy, which is to be transmitted or received by using an ultrasound probe, and a resolution of an ultrasound image composed of raw data. A suitable parameter set may be previously set in the ultrasound apparatus 1000 in correspondence with a photographing part. Therefore, the user may select a photographing part for selecting a plurality of previously set parameter values. Thus, the ultrasound apparatus 1000 may acquire a photographing part which is set in the ultrasound apparatus 1000 in correspondence with the first ultrasound image displayed on the screen. According to an exemplary embodiment, a parameter set which is pre-stored in correspondence with a photographing part may be referred to as a preset.

Moreover, according to an exemplary embodiment, identification information of an organ which the first ultrasound image represents may be stored in a metadata format in a first ultrasound image file. Therefore, the ultrasound apparatus 1000 may acquire the identification of the organ, which the first ultrasound image represents, from metadata of the first ultrasound image displayed on the screen.

In operation S330, the ultrasound apparatus 1000 may display a setting window including at least one recommendation annotation of a plurality of annotations corresponding to the determined photographing part of the object.

A plurality of annotations may be stored in the ultrasound apparatus 1000 in correspondence with a photographing part.

As a user input which selects a menu for setting an annotation in the first ultrasound image is received, the ultrasound apparatus 1000 may acquire a plurality of annotations corresponding to a photographing part, based on a currently set photographing part. The plurality of annotations corresponding to the photographing part may include an annotation which is pre-stored in the ultrasound apparatus 1000 in correspondence with the photographing part.

The at least one recommendation annotation may be an annotation, which is previously input and is generated in the ultrasound apparatus 1000 by the user in correspondence with a second ultrasound image including a photographing part, among a plurality of annotations. The second ultrasound image may denote an ultrasound image in which an annotation is set in the ultrasound apparatus 1000 before the first ultrasound image is displayed.

Moreover, the at least one recommendation annotation may include an annotation of which a set frequency number is high. For example, the at least one recommendation annotation may be an annotation, of which the order is within a predetermined order with respect to the number of times the annotation is set in the ultrasound apparatus 1000, among a plurality of annotations.

Moreover, the at least one recommendation annotation may include an annotation in which the order of generation is fast. For example, the at least one recommendation annotation may be an annotation, of which the order is within a predetermined order with respect to the number of times the annotation is generated in the ultrasound apparatus 1000, among a plurality of annotations.

Moreover, the at least one recommendation annotation may include an arrow image in addition to a text annotation and a body marker. Also, the at least one recommendation annotation may include different kinds of annotations. For example, the ultrasound apparatus 1000 may display a setting window including the body marker and the text annotation.

The ultrasound apparatus 1000 may store a user generation annotation in correspondence with a photographing part. Also, the orders of a plurality of annotations corresponding to a photographing part may be determined in the ultrasound apparatus 1000, based on the number of times an annotation is set or the order in which an annotation is generated.

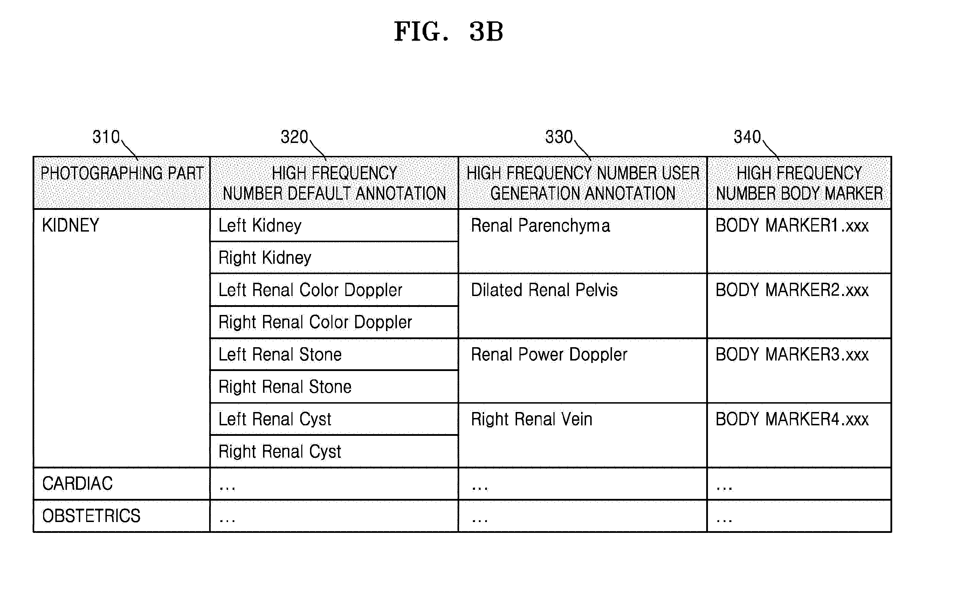

For example, referring to FIG. 3B, a high frequency number default text annotation 320, a high frequency number user generation text annotation 330, and a high frequency number body marker 340 may be stored in the form of a database in the ultrasound apparatus 1000.

A default annotation may not be generated by the user but may be pre-stored in the ultrasound apparatus 1000, or may denote an annotation received from an external ultrasound apparatus 1000. As a user input which sets the default annotation is received, the ultrasound apparatus 1000 may increase the number of times the default annotation is set, in correspondence with identification information of the default annotation.

The user generation annotation may denote an annotation, which is not the same as the default annotation, among a plurality of annotations which are set in an ultrasound image by the user. For example, when the user sets an annotation in an ultrasound image by manually inputting the annotation without selecting a recommendation annotation, the ultrasound apparatus 1000 may determine whether the set annotation is the same as the default annotation or the stored user generation annotation. When the set annotation is not the same as the default annotation or the stored user generation annotation, the ultrasound apparatus 1000 may store the set annotation as a new user generation annotation corresponding to a set photographing part. In this case, the ultrasound apparatus 1000 may store a date and a time, at which the user generation annotation is generated, along with the user generation annotation in correspondence with the user generation annotation.

Moreover, when the set annotation is the same as the default annotation or the stored user generation annotation, the ultrasound apparatus 1000 may increase the number of settings in correspondence with identification information of the default annotation or the stored user generation annotation which is the same as the set annotation.

The ultrasound apparatus 1000 may determine the orders of a plurality of annotations, based on the number of settings. For example, as the number of settings increases, the ultrasound apparatus 1000 may determine the order of a corresponding annotation as a fast order.

Moreover, the ultrasound apparatus 1000 may determine the orders of a plurality of annotations, based on a date and a time at which the user generation annotation is generated. For example, as a generation time becomes earlier, the ultrasound apparatus 1000 may determine the order of a corresponding annotation as a fast order.

Therefore, the ultrasound apparatus 1000 may acquire, as at least one recommendation annotation, a default annotation in which a frequency number of setting is high, a user generation annotation in which a frequency number of setting is high, and a body marker in which a frequency number of setting is high, in correspondence with a photographing part.

Moreover, the ultrasound apparatus 1000 may display a recommendation annotation in descending order of a frequency number of setting, based on a frequency number of setting of the recommendation annotation. Also, the ultrasound apparatus 1000 may display the recommendation annotation in descending order of the order of generation, based on the order in which the recommendation annotation is generated.

FIG. 3 illustrates only a recommendation annotation corresponding to a photographing part. However, a recommendation annotation corresponding to a user and a photographing part may be stored, and a recommendation annotation corresponding to an object and a photographing part may be stored.

In operation S340, the ultrasound apparatus 1000 may receive a user input which selects one from among at least one or more recommendation annotations.

The ultrasound apparatus 1000 may receive a user input which selects one from among at least one or more recommendation annotations in a setting window. The at least one or more recommendation annotations may be displayed in the form of an interface object (for example, a button) in the setting window. Therefore, the ultrasound apparatus 1000 may receive the user input which selects one from among the at least one or more recommendation annotations.

In operation S350, the ultrasound apparatus 1000 may display an image, representing the selected recommendation annotation, on an ultrasound image.

Moreover, as a user input which selects and moves a recommendation annotation displayed on an ultrasound image is received, the ultrasound apparatus 1000 may move a position of the recommendation annotation in the ultrasound image.

Moreover, the ultrasound apparatus 1000 may store an annotation in correspondence with an ultrasound image. For example, the ultrasound apparatus 1000 may store identification information or display position information of an annotation in correspondence with identification information of the ultrasound image. Also, the ultrasound apparatus 1000 may change a pixel value constituting the ultrasound image in order for an annotation to be engraved on the ultrasound image, and store the ultrasound image.

Moreover, according to an exemplary embodiment, the ultrasound apparatus 1000 may provide a recommendation annotation in correspondence with a photographing part of an object, and moreover provide recommendation annotations in correspondence with a photographing part and identification information of a user. Also, the ultrasound apparatus 1000 may provide recommendation annotations in correspondence with a photographing part and identification information of a patient.

For example, users may use different annotations for the same photographing part. Also, a probability that an ultrasound image of the same patient is captured due to the same disease is high. Therefore, the ultrasound apparatus 1000 may acquire at least one recommendation annotation which is pre-stored in correspondence with a photographing part, based on a user of the ultrasound apparatus 1000 and identification information of an object and display a setting window including the acquired at least one recommendation annotation.

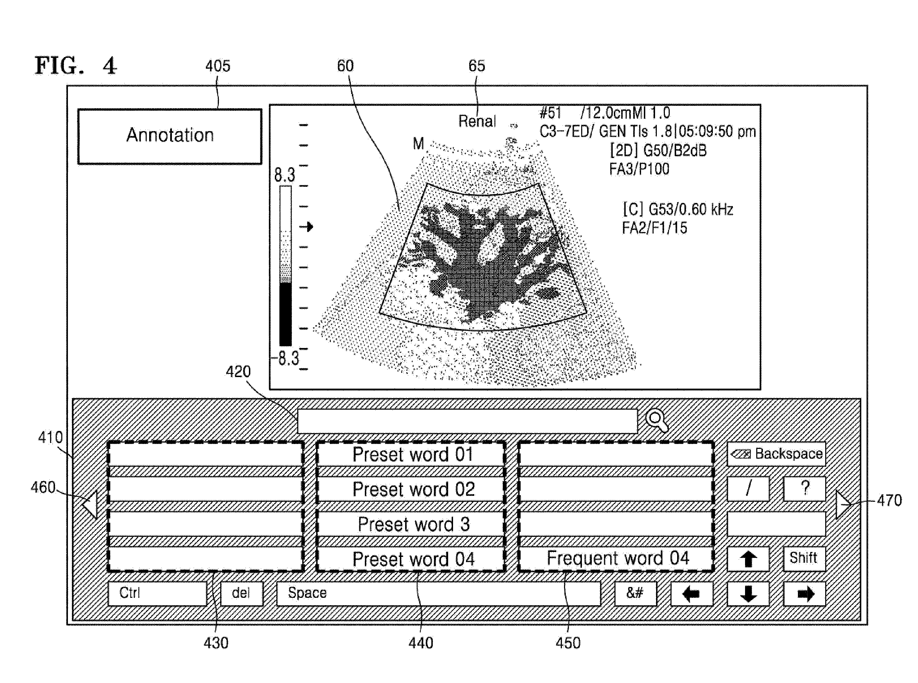

FIG. 4 is a diagram for describing a method in which the ultrasound apparatus 1000 provides a recommendation annotation on the basis of a photographing part, according to an exemplary embodiment.

Referring to FIG. 4, as a user input which selects a button 405 for setting an annotation in an ultrasound image 60 is received, the ultrasound apparatus 1000 may display a setting window 410 including a recommendation text annotation. In this case, the ultrasound apparatus 1000 may display the recommendation text annotation along with a function key.

The ultrasound apparatus 1000 may display a photographing part, set by a user, on a screen. For example, as a user input which selects a liver as a photographing part is received, the ultrasound apparatus 1000 may display a phrase 65 "Renal", representing the liver, on the ultrasound image 60.

The recommendation text annotation may include an annotation 430 which is latest used by the user in correspondence with a currently set photographing part. In this case, the ultrasound apparatus 1000 may display the recommendation text annotation in the order which is latest used.

Moreover, the recommendation text annotation may include an annotation 440 which is previously set as a recommendation annotation in the ultrasound apparatus 1000 by the user in correspondence with the currently set photographing part. In this case, the order in which the previously set annotation 440 is displayed may be set by the user.

Moreover, the recommendation text annotation may include an annotation 450 which is the most used by the user for a certain time in correspondence with the currently set photographing part. In this case, the ultrasound apparatus 1000 may display the recommendation text annotation in the order which is the most used.

As a user input which selects one from among a plurality of recommendation text annotations is received, the ultrasound apparatus 1000 may display a selected annotation on a text box 420.

In a state where the annotation is displayed on the text box 420, as a user input which selects a predetermined key (for example, an enter key) is received, the ultrasound apparatus 1000 may display the annotation, displayed on the text box 420, on the ultrasound image 60. Since the ultrasound apparatus 1000 displays the annotation, displayed on the text box 420, on the ultrasound image 60, the ultrasound apparatus 1000 may store the annotation displayed on the ultrasound image 60 and a position of the annotation in correspondence with the ultrasound image 60.

Moreover, the setting window may include a plurality of movement display buttons 460 and 470 for switching to a setting window for inputting various kinds of annotations. As a user input which selects the movement display buttons 460 and 470 is received, the ultrasound apparatus 1000 may switch a setting window 410, including a recommendation text annotation, to a screen keyboard, an arrow annotation setting window, or a body marker setting window.

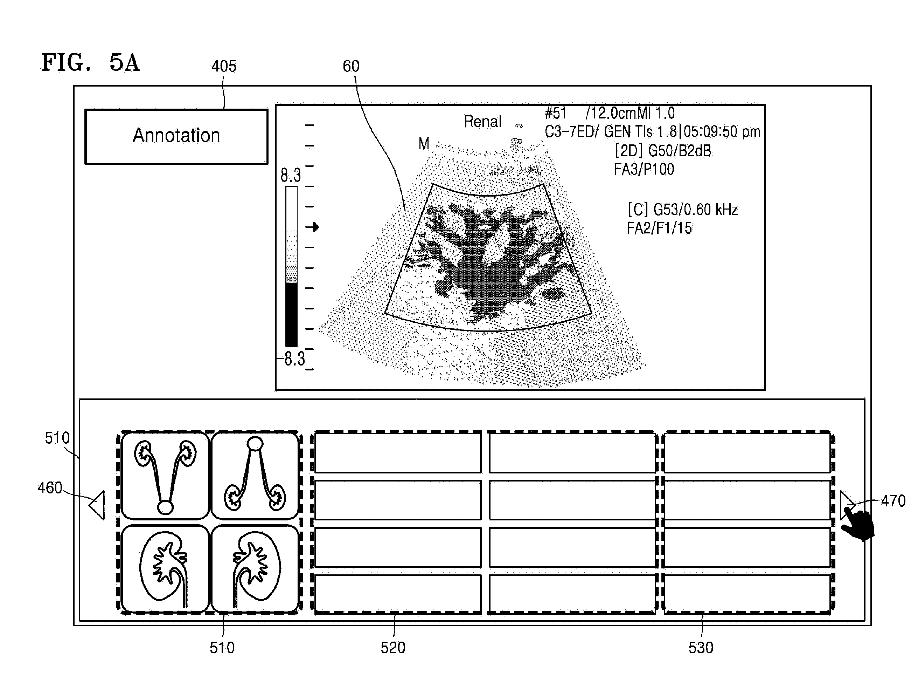

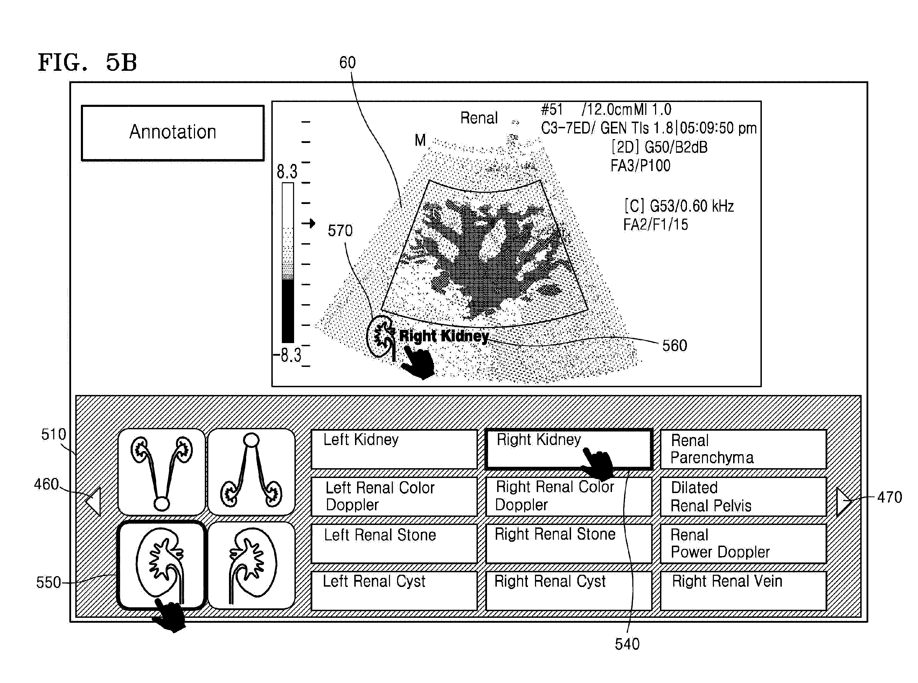

FIGS. 5A and 5B are diagrams for describing a method in which the ultrasound apparatus 1000 provides a recommendation annotation on the basis of a photographing part, according to another exemplary embodiment.

Referring to FIG. 5A, as a user input which selects a button 405 for setting an annotation is received, the ultrasound apparatus 1000 may provide a recommendation annotation, based on a currently set photographing part.

The recommendation annotation may include an annotation in which a frequency number of use is high. A recommendation annotation corresponding to a photographing part may include an annotation which is set in an ultrasound image including the photographing part and in which a frequency number of setting is high. For example, the recommendation annotation may include a high frequency number body marker 510, a high frequency number default text annotation 520, and a high frequency number user generation text annotation 530.

The ultrasound apparatus 1000 may acquire the high frequency number body marker 510, the high frequency number default text annotation 520, and the high frequency number user generation text annotation 530 which correspond to a currently set photographing part. For example, the ultrasound apparatus 1000 may acquire a recommendation annotation, corresponding to a photographing part, from a recommendation annotation database illustrated in FIG. 3B.

The recommendation annotation corresponding to the photographing part is acquired, and the ultrasound apparatus 1000 may display a setting window 510 including the acquired recommendation annotation. The recommendation annotation may be included in the form of a button interface object in the setting window 510.

Referring to FIG. 5B, as a user input which selects one from among at least one or more recommendation annotations is received, the ultrasound apparatus 1000 may display the selected annotation on the ultrasound image 60.

For example, as a user input which selects a button 540 which is included in the setting window 510 and on which Right Kidney is marked is received, the ultrasound apparatus 1000 may display a phrase 560 "Right Kidney" on the ultrasound image 60. Also, as a user input which selects a button 550 on which a body marker 570 representing a right liver is marked is received, the ultrasound apparatus 1000 may display the body marker 570 representing the right liver on the ultrasound image 60.

Moreover, as a user input which selects and drags the body marker 570 representing the right liver or the phrase 560 "Right Kidney" is received, the ultrasound apparatus 1000 may change a position of an annotation, which is selected along a drag region, in the ultrasound image 60. Also, the ultrasound apparatus 1000 may store an annotation displayed on the ultrasound image 60 and a position of the annotation in correspondence with the ultrasound image 60. Also, the ultrasound apparatus 1000 may store the ultrasound image 60 including an annotation.

Therefore, annotations suitable for a diagnosis situation are received, and thus, the user sets an annotation in an ultrasound image through one-time selection even without manually inputting an annotation. Also, by displaying different kinds of annotations on one setting window, the user selects different kinds of annotations even without changing a setting window.

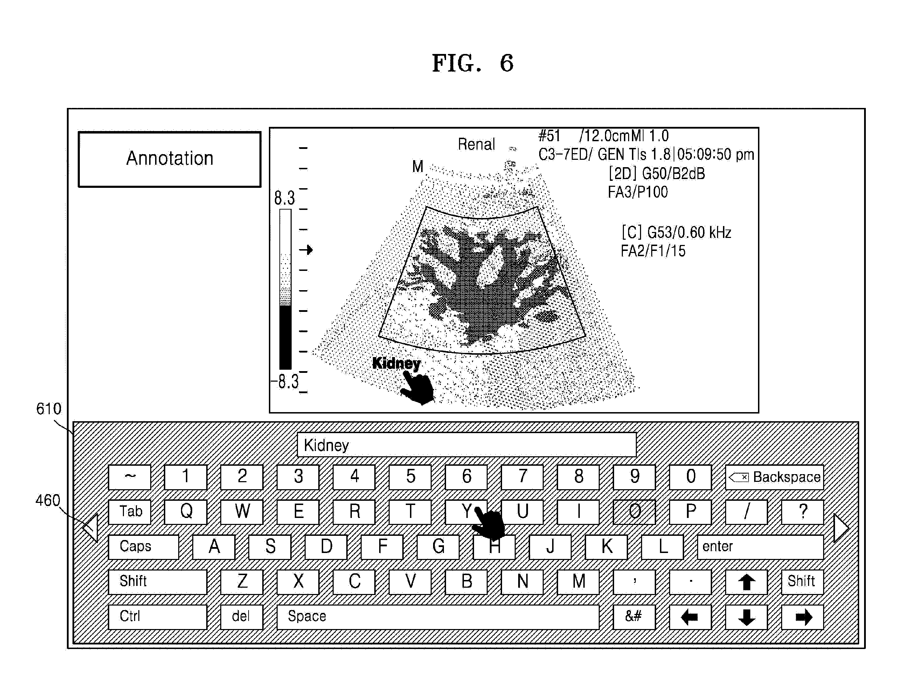

FIG. 6 is a diagram for describing a method in which the ultrasound apparatus 1000 provides a screen keyboard, according to an exemplary embodiment.

Referring to FIG. 6, the ultrasound apparatus 1000 may switch the recommendation annotation setting window 510, illustrated in FIG. 5B, to a screen keyboard 610.

As a user input which selects the movement display buttons 460 and 470 included in the setting window 510 is received, the ultrasound apparatus 1000 may delete the setting window 510 and display the screen keyboard 610 in a region where the setting window 510 was displayed.

Moreover, the screen keyboard 610 may include the movement display buttons 460 and 470 for switching to a setting window for inputting a different kind of annotation.

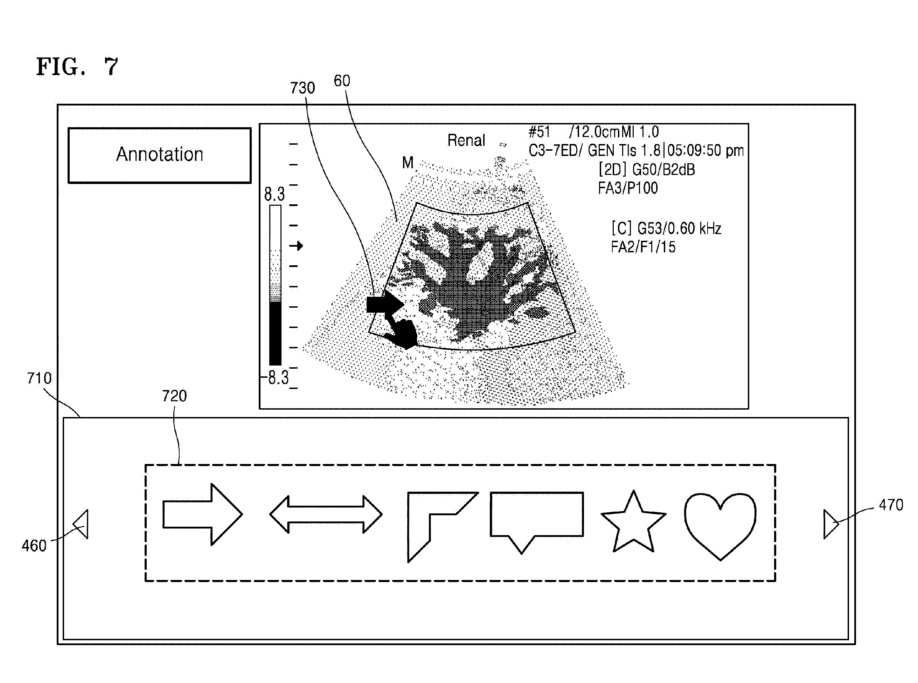

FIG. 7 is a diagram for describing a method in which the ultrasound apparatus 1000 provides a recommendation annotation image, according to an exemplary embodiment.

Referring to FIG. 7, the ultrasound apparatus 1000 may provide a recommendation annotation image such as an arrow in addition to a body marker. In this case, the ultrasound apparatus 1000 may provide a recommendation annotation image in descending order of the number of use for a certain time.

As a user input which selects the movement display buttons 460 and 470 included in the setting window 510 illustrated in FIG. 5B is received, the ultrasound apparatus 1000 may switch the setting window 510 to a setting window 710 including a recommendation annotation image 720.

The ultrasound apparatus 1000 may acquire the recommendation annotation image 720 which is stored in correspondence with a photographing part set in the ultrasound apparatus 1000. The recommendation annotation image 720 which is stored in correspondence with the photographing part may be an annotation image which is set at a high frequency number in an ultrasound image including the photographing part.

As a user input which selects one image from the recommendation annotation image 720 which is stored in correspondence with the photographing part is received, the ultrasound apparatus 1000 may display a selected annotation image 730 on the ultrasound image 60.

Moreover, the setting window 710 including the recommendation annotation image 720 may include the movement display buttons 460 and 470 for switching to a setting window for inputting a different kind of annotation.

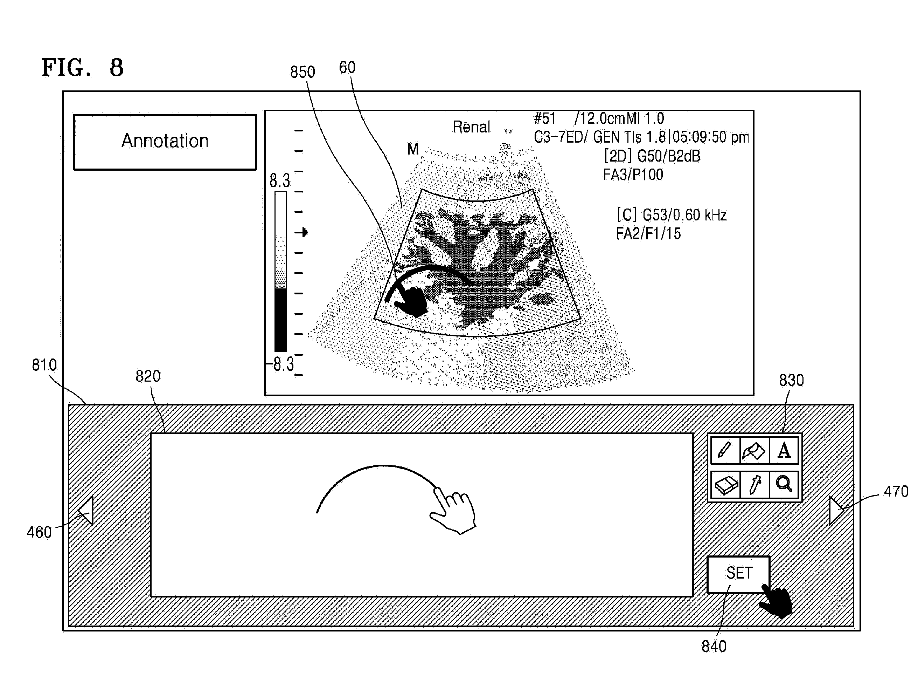

FIG. 8 is a diagram for describing a method in which the ultrasound apparatus 1000 provides an interface for generating an annotation image, according to an exemplary embodiment.

Referring to FIG. 8, the ultrasound apparatus 1000 may provide a setting window 810 for generating an annotation image. The setting window 810 for generating the annotation image may include a picture window 820 for displaying an image which is generated according to a user input and a tool menu 830 for selecting a tool for drawing an image.

As a user input which selects the movement display buttons 460 and 470 included in the setting window 710 of FIG. 7 is received, the ultrasound apparatus 1000 may switch the setting window 710, including the recommendation annotation image 720, to the setting window 810 for generating the annotation image.

As a user input which draws an image in the picture window 820 is received, the ultrasound apparatus 1000 may display an image in the picture window 820, based on a region indicated by a cursor or a touched region. Also, as a user input which selects the setting button 840 in the setting window 810 is received, the ultrasound apparatus 1000 may generate an image, displayed on the picture window 820, as an annotation image 850. Also, the ultrasound apparatus 1000 may display the generated annotation image 850 on the ultrasound image 60.

Moreover, the setting window 810 for generating the annotation image may include the movement display buttons 460 and 470 for switching to a setting window for inputting a different kind of annotation.

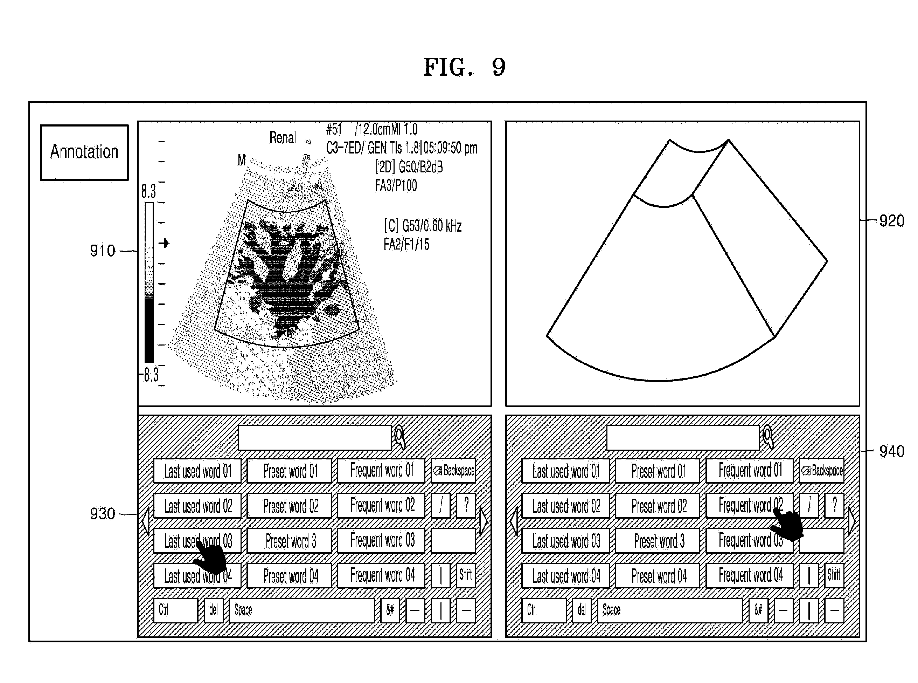

FIG. 9 is a diagram for describing a method in which the ultrasound apparatus 1000 provides a setting window corresponding to a plurality of ultrasound images, according to an exemplary embodiment.

Referring to FIG. 9, the ultrasound apparatus 1000 may display a plurality of ultrasound images on one screen. Also, the ultrasound apparatus 1000 may display a setting window for setting an annotation in each ultrasound image in correspondence with each ultrasound image.

For example, the ultrasound apparatus 1000 may display two ultrasound images. The two ultrasound images may a 2D image 910 and a 3D image 920 of the same photographing part of the same object. Also, the two ultrasound images may be a B mode image and a Doppler image of the same photographing part of the same object.

Moreover, the ultrasound apparatus 1000 may display two setting windows 930 and 940 for setting an annotation in two the ultrasound images 910 and 920 in correspondence with the ultrasound images 910 and 920.

In this case, the ultrasound apparatus 1000 may acquire a recommendation annotation which is to be added to each setting window, based on a photographing part and a kind of an ultrasound image. For example, when a photographing part is a liver and a first ultrasound image is a B mode image, the ultrasound apparatus 1000 may display "Right Kidney" as a recommendation annotation corresponding to the first ultrasound image. When a second ultrasound image is a Doppler image, the ultrasound apparatus 1000 may display "Right Renal Color Doppler" as a recommendation annotation of the second ultrasound image.

Moreover, the ultrasound apparatus 1000 may simultaneously activate setting windows of respective ultrasound images, or may activate only one setting window at a time.

For example, as a user input which selects a setting window 930 corresponding to the 2D image 910 is received, the ultrasound apparatus 1000 may activate a plurality of recommendation annotation buttons in the setting window 930. In this case, the ultrasound apparatus 1000 may deactivate a recommendation annotation button in the setting window 940 corresponding to the 3D image 920.

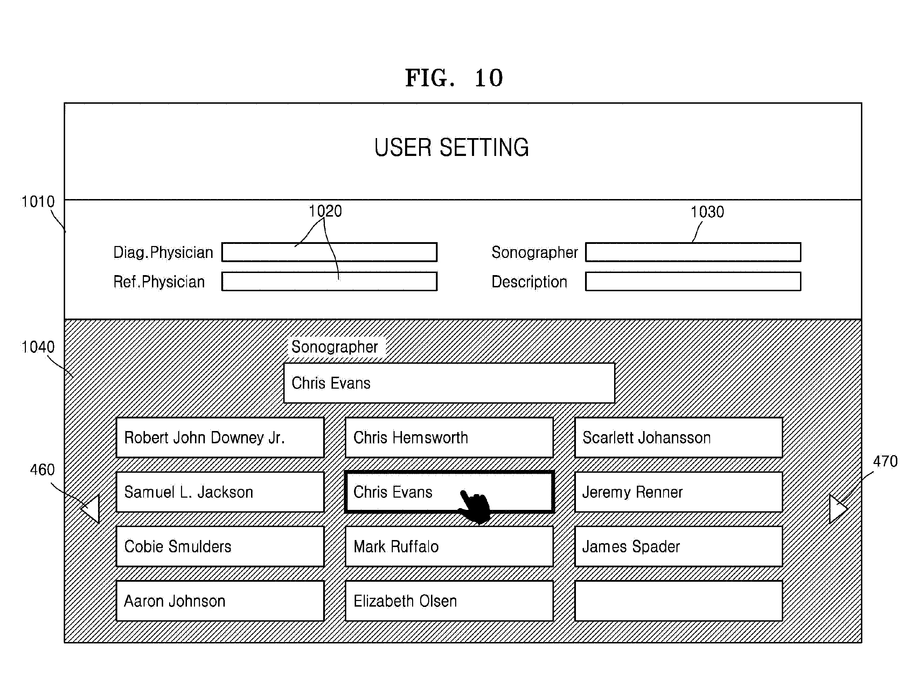

FIG. 10 is a diagram for describing a method in which the ultrasound apparatus 1000 provides recommendation data corresponding to an input field, according to an exemplary embodiment.

For example, the ultrasound apparatus 1000 may display a page 1010 for inputting user information. The page 1010 for inputting the user information may include an input field 1020 for inputting data to an identification information item of a diagnosis and an input field 1030 for inputting data to an identification information item of a sonographer.

As a user input which selects the input field 1030 corresponding to the identification information item of the sonographer is received, the ultrasound apparatus 1000 may acquire recommendation identification information which is pre-stored in correspondence with the identification information item of the sonographer. Since the recommendation identification information is acquired, the ultrasound apparatus 1000 may display a setting window 1040 including the acquired recommendation identification information.

The recommendation identification information, which is pre-stored in correspondence with the identification information item of the sonographer, may be identification information of the sonographer which is generated by previously inputting the sonographer to the ultrasound apparatus 1000. A user input which stores identification information of the sonographer is received, and when the input identification information of the sonographer is identification information which is not pre-stored, the ultrasound apparatus 1000 may store the input identification information of the sonographer as new identification information of the sonographer in correspondence with the identification information item of the sonographer.

Moreover, the recommendation identification information which is pre-stored in correspondence with the identification information item of the sonographer may be identification information of the sonographer, in which the number of times identification information is stored for a certain period is large, among pieces of pre-stored identification information of the sonographer. For example, when a user input which stores identification information of the sonographer in correspondence with the identification information item of the sonographer is received, the ultrasound apparatus 1000 may calculate the number of storages of the stored identification information of the sonographer. Therefore, the ultrasound apparatus 1000 may determine data, in which the number of inputs is large in correspondence with a certain item, as recommendation data about the certain item.

As a user input which selects one from among pieces of displayed recommendation identification information is received, the ultrasound apparatus 1000 may set the selected recommendation identification information in the input field 1030 corresponding to the identification information item of the sonographer.

Moreover, as a user input which selects the movement display buttons 460 and 470 included in the setting window 1040 is received, the ultrasound apparatus 1000 may display other pieces of recommendation identification information.

Therefore, although data is not manually input each time the data is input, a user simply selects and inputs data which was input by the user.

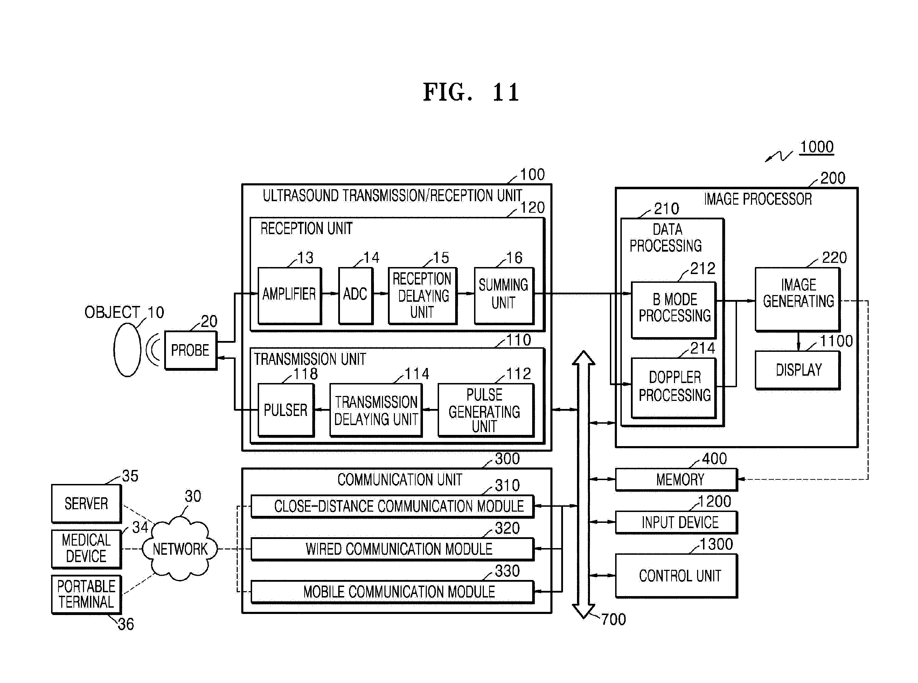

FIG. 11 is a block diagram of the ultrasound apparatus 1000 according to another exemplary embodiment.

Referring to FIG. 11, the ultrasound apparatus 1000 may further include a probe 20, an ultrasound transceiver 100, an image processor 200, a communication module 300, and a memory 400, in addition to the display unit 1100, the user input unit 1200, and the control unit 1300. The above-described elements may be connected to each other through a bus 700.

The ultrasound apparatus 1000 may be implemented in a portable type as well as a cart type. Examples of the portable ultrasound apparatus 1000 may include a picture archiving and communication system (PACS) viewer, a smartphone, a laptop computer, a personal digital assistant (PDA), and a tablet personal computer (PC), but are not limited thereto.

The probe 20 transmits ultrasound waves to an object 10 in response to a driving signal applied by the ultrasound transceiver 100 and receives echo signals reflected by the object 10. The probe 20 includes a plurality of transducers, and the plurality of transducers oscillate in response to electric signals and generate acoustic energy, that is, ultrasound waves. Furthermore, the probe 20 may be connected to the main body of the ultrasound apparatus 1000 by wire or wirelessly, and the ultrasound apparatus 1000 may include a plurality of the probes 20 depending on an implementation type.

A transmitter 110 supplies a driving signal to the probe 20. The transmitter 110 includes a pulse generator 112, a transmission delaying unit 114, and a pulser 116. The pulse generator 112 generates pulses for forming transmission ultrasound waves based on a predetermined pulse repetition frequency (PRF), and the transmission delaying unit 114 delays the pulses by delay times necessary for determining transmission directionality. The pulses which have been delayed correspond to a plurality of piezoelectric vibrators included in the probe 20, respectively. The pulser 116 applies a driving signal (or a driving pulse) to the probe 20 based on timing corresponding to each of the pulses which have been delayed.

A receiver 120 generates ultrasound data by processing echo signals received from the probe 20. The receiver 120 may include an amplifier 122, an analog-to-digital converter (ADC) 124, a reception delaying unit 126, and a summing unit 128. The amplifier 122 amplifies echo signals in each channel, and the ADC 124 performs analog-to-digital conversion with respect to the amplified echo signals. The reception delaying unit 126 delays digital echo signals output by the ADC 124 by delay times necessary for determining reception directionality, and the summing unit 128 generates ultrasound data by summing the echo signals processed by the reception delaying unit 126.

The image processor 200 generates an ultrasound image by scan-converting ultrasound data generated by the ultrasound transceiver 100 and displays the ultrasound image. The ultrasound image may be not only a grayscale ultrasound image obtained by scanning an object in an amplitude (A) mode, a brightness (B) mode, and a motion (M) mode, but also a motion of an object may be displayed as a Doppler image. The Doppler image may be a blood flow Doppler image showing flow of blood (also referred to as a color Doppler image), a tissue Doppler image showing a movement of tissue, or a spectral Doppler image showing a moving speed of an object as a waveform.

A B mode processor 212 extracts B mode components from ultrasound data and processes the B mode components. An image generator 220 may generate an ultrasound image indicating signal intensities as brightness based on the extracted B mode components 212.

Similarly, a Doppler processor 214 may extract Doppler components from ultrasound data, and the image generator 220 may generate a Doppler image indicating a movement of an object as colors or waveforms based on the extracted Doppler components.

According to an embodiment, the image generator 220 may generate a three-dimensional (3D) ultrasound image via volume-rendering with respect to volume data and may also generate an elasticity image by imaging deformation of the object 10 due to pressure. Furthermore, the image generator 220 may display various pieces of additional information in an ultrasound image by using text and graphics. In addition, the generated ultrasound image may be stored in the memory 400.

In addition, the ultrasound diagnosis apparatus 1000 may include two or more displays 1100 according to embodiments.

The communication module 300 is connected to a network 30 by wire or wirelessly to communicate with an external device or a server. The communication module 300 may exchange data with a hospital server or another medical apparatus in a hospital, which is connected thereto via a PACS. Furthermore, the communication module 300 may perform data communication according to the digital imaging and communications in medicine (DICOM) standard.

The communication module 300 may transmit or receive data related to diagnosis of an object, e.g., an ultrasound image, ultrasound data, and Doppler data of the object, via the network 30 and may also transmit or receive medical images captured by another medical apparatus, e.g., a computed tomography (CT) apparatus, a magnetic resonance imaging (MRI) apparatus, or an X-ray apparatus. Furthermore, the communication module 300 may receive information about a diagnosis history or medical treatment schedule of a patient from a server and utilizes the received information to diagnose the patient. Furthermore, the communication module 300 may perform data communication not only with a server or a medical apparatus in a hospital, but also with a portable terminal of a medical doctor or patient.

The communication module 300 is connected to the network 30 by wire or wirelessly to exchange data with a server 32, a medical apparatus 34, or a portable terminal 36. The communication module 300 may include one or more components for communication with external devices. For example, the communication module 1300 may include a local area communication module 310, a wired communication module 320, and a mobile communication module 330.

The local area communication module 310 refers to a module for local area communication within a predetermined distance. Examples of local area communication techniques according to an embodiment may include, but are not limited to, wireless LAN, Wi-Fi, Bluetooth, ZigBee, Wi-Fi Direct (WFD), ultra wideband (UWB), infrared data association (IrDA), Bluetooth low energy (BLE), and near field communication (NFC).

The wired communication module 320 refers to a module for communication using electric signals or optical signals. Examples of wired communication techniques according to an embodiment may include communication via a twisted pair cable, a coaxial cable, an optical fiber cable, and an Ethernet cable.

The mobile communication module 330 transmits or receives wireless signals to or from at least one selected from a base station, an external terminal, and a server on a mobile communication network. The wireless signals may be voice call signals, video call signals, or various types of data for transmission and reception of text/multimedia messages.

The memory 400 stores various data processed by the ultrasound apparatus 1000. For example, the memory 400 may store medical data related to diagnosis of an object, such as ultrasound data and an ultrasound image that are input or output, and may also store algorithms or programs which are to be executed in the ultrasound apparatus 1000.

The memory 400 may be any of various storage media, e.g., a flash memory, a hard disk drive, EEPROM, etc. Furthermore, the ultrasound apparatus 1000 may utilize web storage or a cloud server that performs the storage function of the memory 400 online.

The user input unit 1200 may further include various input means such as an electrocardiogram measurement module, a breath measurement module, a voice recognition sensor, a gesture recognition sensor, a fingerprint recognition sensor, an iris recognition sensor, a depth sensor, a distance sensor, etc.

All or some of the probe 20, the ultrasound transceiver 100, the image processor 200, the communication module 300, the memory 400, the user input unit 1200, and the controller 1300 may be implemented as software modules. However, embodiments of the present invention are not limited thereto, and some of the components stated above may be implemented as hardware modules. Furthermore, at least one selected from the ultrasound transceiver 100, the image processor 200, and the communication module 300 may be included in the controller 1300. However, embodiments of the present invention are not limited thereto.

The method according to the exemplary embodiments may be implemented as computer readable codes in a computer readable medium. The computer readable recording medium may include a program instruction, a local data file, a local data structure, or a combination thereof. The computer readable recording medium may be specific to exemplary embodiments of the invention or commonly known to those of ordinary skill in computer software. The computer readable recording medium includes all types of recordable media in which computer readable data are stored. Examples of the computer readable recording medium include a magnetic medium, such as a hard disk, a floppy disk and a magnetic tape, an optical medium, such as a CD-ROM and a DVD, a magneto-optical medium, such as a floptical disk, and a hardware memory, such as a ROM, a RAM and a flash memory, specifically configured to store and execute program instructions. Furthermore, the computer readable recording medium may be implemented in the form of a transmission medium, such as light, wire or waveguide, to transmit signals which designate program instructions, local data structures and the like. Examples of the program instruction include machine code, which is generated by a compiler, and a high level language, which is executed by a computer using an interpreter and so on.

It should be understood that the exemplary embodiments described therein should be considered in a descriptive sense only and not for purposes of limitation. Descriptions of features or aspects within each exemplary embodiment should typically be considered as available for other similar features or aspects in other exemplary embodiments.

While one or more exemplary embodiments have been described with reference to the figures, it will be understood by those of ordinary skill in the art that various changes in form and details may be made therein without departing from the spirit and scope as defined by the following claims.

## Claims

1. An ultrasound apparatus comprising:
a user input unit that receives a user input which selects a menu for setting an annotation in a first ultrasound image displayed on a screen of the ultrasound apparatus;
a control unit that determines a photographing part of an object in the first ultrasound image; and
a display unit that displays a setting window including at least one recommendation annotation among a plurality of annotations corresponding to the determined photographing part of the object,
wherein,
the user input unit receives a user input which selects one from the at least one recommendation annotation, and the display unit displays an image, representing the selected recommendation annotation, on the first ultrasound image, and
the at least one recommendation annotation is an annotation which is previously input by the user and is generated in the ultrasound apparatus in correspondence with a second ultrasound image including the photographing part.

2. The ultrasound apparatus of claim 1, wherein the at least one recommendation annotation comprises different kinds of annotations.

3. The ultrasound apparatus of claim 1, wherein the at least one recommendation annotation is an annotation which is previously set in the ultrasound apparatus by the user in correspondence with the photographing part.

4. The ultrasound apparatus of claim 1, wherein the at least one recommendation annotation comprises at least one selected from a phrase, a body marker, and an arrow.

5. The ultrasound apparatus of claim 1, further comprising a storage unit that stores an order of the plurality of annotations with respect to the number of settings,
wherein the at least one recommendation annotation is an annotation, in which the order is within a predetermined order, among the plurality of annotations.

6. The ultrasound apparatus of claim 1, further comprising a storage unit that stores an order of the plurality of annotations with respect to an order in which the plurality of annotations are generated in the ultrasound apparatus,
wherein the at least one recommendation annotation is an annotation, in which the order is within a predetermined order, among the plurality of annotations.

7. The ultrasound apparatus of claim 1, wherein,
the setting window comprises a movement display button that switches the setting window to a screen keyboard including a plurality of keys, and
when a user input which selects the movement display button is received, the display unit switches the setting window to the screen keyboard and displays the screen keyboard.

8. The ultrasound apparatus of claim 1, wherein,
the first ultrasound image comprises two different ultrasound images, and
the setting window comprises two setting windows respectively corresponding to the two different ultrasound images.

9. An information input method comprising:
receiving a user input which selects a menu for setting an annotation in a first ultrasound image displayed on a screen of an ultrasound apparatus;
determining a photographing part of an object in the first ultrasound image;
displaying a setting window including at least one recommendation annotation among a plurality of annotations corresponding to the determined photographing part of the object;
receiving a user input which selects one from the at least one recommendation annotation; and
displaying an image, representing the selected recommendation annotation, on the first ultrasound image,
wherein the at least one recommendation annotation is an annotation which is previously input by the user and is generated in the ultrasound apparatus in correspondence with a second ultrasound image including the photographing part.

10. The information input method of claim 9, wherein the at least one recommendation annotation comprises different kinds of annotations.

11. The information input method of claim 9, wherein the at least one recommendation annotation is an annotation which is previously set in the ultrasound apparatus by the user in correspondence with the photographing part.

12. The information input method of claim 9, wherein
an order of the plurality of annotations is determined based on the number of settings in the ultrasound apparatus, and
the at least one recommendation annotation is an annotation, in which the order is within a predetermined order, among the plurality of annotations.

13. The information input method of claim 9, wherein
an order of the plurality of annotations is determined based on an order in which the plurality of annotations are generated in the ultrasound apparatus, and
the at least one recommendation annotation is an annotation, in which the order is within a predetermined order, among the plurality of annotations.

14. The information input method of claim 9, wherein
the setting window comprises a movement display button that switches the setting window to a screen keyboard including a plurality of keys, and
the information input method further comprises, when a user input which selects the movement display button is received, switching the setting window to the screen keyboard and displaying the screen keyboard.

15. The information input method of claim 9, wherein
the first ultrasound image comprises two different ultrasound images, and the setting window comprises two setting windows respectively corresponding to the two different ultrasound images.
